# EUROPÄISCHE PATENTSCHRIFT

(11) **EP 0 616 611 B1**
(45) Veröffentlichungstag und Bekanntmachung des Hinweises auf die Patenterteilung: **02.05.1997**
(21) Anmeldenummer: 92923815.2
(22) Anmeldetag: 27.11.1992
(51) Int. Cl.: C07H 15/04

(54) **VERFAHREN ZUR HERSTELLUNG VON NIEDRIGALKYLOLIGOGLUCOSIDEN**
METHOD OF MANUFACTURING LOWER-ALKYL OLIGOGLUCOSIDES
PROCEDE DE FABRICATION D'OLIGOGLUCOSIDES D'ALKYLE INFERIEUR

(30) Priorität: 06.12.1991 DE 4140334
(43) Veröffentlichungstag der Anmeldung: 28.09.1994
(73) Patentinhaber: Henkel Kommanditgesellschaft auf Aktien, 40191 Düsseldorf (DE)
(72) Erfinder: SCHULZ, Paul, D-5600 Wuppertal 1 (DE); ESSER, Herbert, D-5210 Troisdorf 14 (DE); ESKUCHEN, Rainer, D-4000 Düsseldorf 13 (DE)
(86) Internationale Anmeldenummer: EP9202748
(87) Internationale Veröffentlichungsnummer: WO9311142

(56) Entgegenhaltungen:
- EP-A- 0 301 298
- EP-A- 0 319 616
- EP-A- 0 357 969

## Beschreibung

### Gebiet der Erfindung

Die Erfindung betrifft ein Verfahren zur Herstellung von Niedrigalkyloligoglucosiden, bei dem man Glucosesirup bei erhöhter Temperatur über einen Inline-Mischer einer Mischung aus einem kurzkettigen Alkohol und einem sauren Katalysator zudosiert, das Reaktionswasser azeotrop abdestilliert und die Reaktionsmischung einer Nachreaktion unterwirft.

### Stand der Technik

Oberflächenaktive langkettige Alkyloligoglucoside sind als Rohstoffe für die Herstellung von Waschmitteln bereits seit langem bekannt. Ihre Herstellung erfolgt üblicherweise durch säurekatalysierte Acetalisierung von Glucose mit langkettigen Fettalkoholen. Bei den als "Umacetalisierungsverfahren" oder im Sonderfall als "Butanol-Route" bekannten Verfahren wird im ersten Schritt die Glucose mit einem kurzkettigen Alkohol, z.B. Butanol zum entsprechenden Glucosid umgesetzt, welches dann in einer zweiten Stufe einer Umacetalisierung mit einem Fettalkohol unterworfen wird. Niedrigalkyloligoglucoside wie beispielsweise Butylglucoside stellen somit wichtige Zwischenprodukte für die Herstellung von langkettigen Alkyloligoglucosiden dar; sie selbst finden darüber hinaus als Lösungsvermittler und Emulgatoren Verwendung.

Aus der Europäischen Patentanmeldung **EP 0 319 616 A1** ist ein Verfahren zur Herstellung von Niedrigalkyloligoglykosiden bekannt, bei dem man eine wäßrige Zuckerlösung mit einem kurzkettigen Alkohol vermischt, der homogenen Lösung einen sauren Katalysator zusetzt und die Mischung anschließend bei 60 bis 200, vorzugsweise 80 bis 150°C zur Reaktion bringt, wobei enthaltenes Wasser azeotrop abdestilliert wird.

In der Internationalen Patentanmeldung **WO 90/1489** wird die Umsetzung von Glucosesirupen mit einem DP 1-Gehalt (d. h. Monomeranteil) von weniger als 90 Gew.-% mit Butanol beschrieben. Die Acetalisierung wird unter intensivem Rühren im wäßrigen System bei Temperaturen oberhalb von 125°C und Drücken von 4 bis 10 bar durchgeführt, wobei die Reaktionsmischung über einen Inline-Dispergator im Kreis gepumpt wird. Die Zudosierung des Sirups erfolgt unmittelbar in den Reaktor. Nach Beendigung der Zugabe wird die Reaktionsmischung einer Nachreaktion unterworfen, um eine vollständige Abreaktion der Glucose sicherzustellen.

Die geschilderten Verfahren weisen Nachteile auf, die ihre technische Verwertbarkeit stark einschränken: dosiert man den Glucosesirup zu rasch in die Mischung aus Alkohol und saurem Katalysator, findet auch bei intensivem Rühren keine ausreichende Dispergierung statt. Der Glucosesirup durchläuft vielmehr unter Wasserabgabe ein klebrig-viskoses Zwischenstadium, das zu praktisch nicht mehr redispergierbaren Agglomeraten führt. Diese zeigen die Tendenz, sich vorzugsweise an der Rührereinheit abzuscheiden, sie zu verkleben und im Extremfall sogar zu blockieren. Zudem bleibt gewöhnlich ein weiterer Teil des nicht dispergierten, verklumpten Einsatzstoffs an der beheizten Reaktorwandung haften, wodurch es zu partieller Verkohlung kommen kann. Es ist selbstverständlich, daß die Acetalisierungsreaktion zwischen Glucose und Alkohol bei dieser Vorgehensweise nicht optimal ablaufen kann: die Umsetzung findet im wesentlichen nicht während der Zudosierung, sondern erst im Verlauf der Nachreaktion statt, die aus diesem Grunde ganz erheblich zeitlich ausgedehnt werden muß.

Üblicherweise umgeht man das geschilderte Problem, indem man den Glucosesirup langsam, d. h. mit einer solchen Geschwindigkeit zudosiert, daß das Verklumpen nicht auftritt, sondern ein einphasiges, homogenes System vorliegt bzw. eine mehr oder minder feine Dispersion entsteht, in der die Acetalisierung leicht statfinden kann. Auf diese Weise kann zwar die Nachreaktionszeit auf ein wirtschaftlich vertretbares Maß verkürzt werden, die Zudosierungszeit verlängert sich jedoch entscheidend, so daß in Summe ebenfalls wieder lange Kesselbelegungszeiten resultieren.

Ein weiterer Nachteil der langen Reaktionszeiten besteht darin, daß die Reaktionsendprodukte vergleichsweise hohe Gehalte an Polyglucose und anderen unerwünschten Nebenprodukten, wie beispielsweise Dialkylethern, aufweisen und zudem durch die hohe thermische Belastung stark verfärbt sein können.

Die Aufgabe der Erfindung bestand nun darin, ein verbessertes Verfahren zur Herstellung von Niedrigalkyloligoglucosiden zu entwickeln, das frei von den geschilderten Nachteilen ist.

### Beschreibung der Erfindung

Gegenstand der Erfindung ist ein Verfahren zur Herstellung von Niedrigalkyloligoglucosiden, das sich dadurch auszeichnet, daß man
a) wäßrigen Glucosesirup bei erhöhten Temperaturen über einen Inline-Mischer einer Mischung aus einem Niedrigalkohol und einem sauren Katalysator zudosiert,
b) im Reaktionsgemisch enthaltenes sowie freigesetztes Wasser kontinuierlich azeotrop abdestilliert sowie
c) die Reaktionsmischung nach Beendigung der Zugabe an Glucosesirup einer Nachreaktion unterwirft.

Überraschenderweise wurde gefunden, daß sich auch hochabgebauter Glucosesirup problemlos in der Reaktionsmischung dispergieren läßt, wenn er über einen Inline-Mischer zudosiert wird. Auf diese Weise kann die Gesamtreaktionszeit, d. h. die Summe aus Dosierzeit und Nachreaktonszeit, entscheidend verkürzt werden. Es werden Niedrigalkyloligoglucoside erhalten, die sich gegenüber den Produkten des Stands der Technik durch eine hellere Farbe und einen signifikant verminderten Anteil an Nebenprodukten auszeichnen.

Unter **Glucosesirup** ist ein hochabgebautes wäßriges Stärkeprodukt zu verstehen, das einem Feststoffanteil von 50 bis 85, vorzugsweise 70 bis 80 Gew.-% - bezogen auf den Sirup - sowie einen DP-Grad 1 (d.h. einen monomeren Glucoseanteil) von 90 bis 100 Gew.-% - bezogen auf den Feststoffanteil - aufweist.

**Inline-Mischer**, die im Sinne des erfindungsgemäßen Verfahrens eingesetzt werden können, stellen hochtourige Stator-Rotor-Dispergatoren mit geeignet strukturierter Scherorgan-Oberfläche dar. Inline-Mischer, die sich in besonderer Weise für die rasche Dispergierung des Glucosesirups in der Reaktionsmischung eignen, weisen Schergefälle von 10⁴ bis 10⁶ s⁻¹ auf. Infolge geeigneter Strukturierung der Rotor- und Statorringe, z. B. durch Verzahnung, Riffelung oder Perforation, erhält das Scherfeld einen zeitlich pulsierenden Verlauf der die Dispergierwirkung erhöht. Als optimal haben sich Inline-Mischer erwiesen, die mit einer mittleren Scherimpulszahl pro Volumeneinheit von 10⁵ bis 10⁸ l⁻¹ arbeiten.

Die Inline-Mischer werden vorzugsweise außerhalb des Reaktionsgefäßes in den Flüssigkeitskreislauf geschaltet. Es ist jedoch ebenfalls möglich, die Mischsysteme in einem Strömungs-Leitrohr innerhalb des Reaktors zu betreiben.

Unter **Niedrigalkohole** sind im Sinne des erfindungsgemäßen Verfahrens primäre Alkohol der Formel (I) zu verstehen,

**R**^{**1**}**-OH** (I)

in der R¹ für einen linearen oder verzweigten Alkylrest mit 3 bis 8 Kohlenstoffatomen steht. Typische Beispiele sind n-Propanol, Isopropylalkohol, i-Butanol, sec.-Butanol, tert.-Butanol, Pentanol, Hexanol, Heptanol, n-Octanol und 2-Ethylhexanol. Vorzugsweise wird n-Butanol eingesetzt.

Die Acetalisierung wird in Gegenwart **saurer Katalysatoren** durchgeführt. Typische Beispiele hierfür sind Methansulfonsäure, Butansulfonsäure und Sulfobernsteinsäure. Bevorzugt wird p-Toluolsulfonsäure eingesetzt.

Zur Verlagerung des Acetalisierungsgleichgewichtes auf die Seite der Glucoside empfiehlt es sich, den Niedrigalkohol in erheblichen Überschuß vorzulegen. Typischerweise können Glucose und Niedrigalkohol in einem molaren Verhältnis von 1 : 3 bis 1 : 10, vorzugsweise 1 : 6 bis 1 : 8 eingesetzt werden.

Die Einsatzmenge des sauren Katalysators kann 3 * 10⁻³ bis 2 * 10⁻², vorzugsweise 5 * 10⁻³ bis 1 * 10⁻² mol pro mol Glucose betragen.

Die Alkoholyse der Glucose stellt vorzugsweise eine Butanolyse dar. Dabei empfiehlt es sich, zunächst eine Lösung des sauren Katalysators im überschüssigen Niedrigalkohol herzustellen, die Mischung auf die Reaktionstemperatur von 100 bis 115, vorzugsweise 108 bis 113°C zu erhitzen und den Glucosesirup kontinuierlich über einen Zeitraum von 0,1 bis 3, vorzugsweise 1 bis 2 h mit einer solchen Geschwindigkeit zuzudosieren, daß eine feinteilige Dispersion entsteht. Unter "feinteilig" ist in diesem Zusammenhang zu verstehen, daß die Dispersion frei von deutlich sichtbaren, klebrigen Agglomeraten ist.

In einer bevorzugten Ausführungsform des erfindungsgemäßen Verfahrens wird die Mischung aus Glucosesirup, Niedrigalkohol und Katalysator im Kreislauf über einen Fallfilmverdampfer geführt. Die hohe Stoffaustauschfläche, die geringe Filmdicke und die große Durchflußgeschwindigkeit gewährleisten eine besonders schonende Wärmeübertragung sowie eine sehr effiziente Wasserverdampfung aus der Reaktionsmischung, was sich in einer hohen Farbqualität der Reaktionsendprodukte niederschlägt.

Zur Einstellung des Gleichgewichtes ist es erforderlich, sowohl das Wasser aus dem Glucosesirup, als auch das Reaktionswasser möglichst rasch und möglichst vollständig aus der Reaktionsmischung zu entfernen. Dies geschieht vorzugsweise mittels einer handelsüblichen Destillationskolonne, über die ein azeotropes Gemisch aus Wasser und Niedrigalkohol abgezogen werden kann. Im Fall von Butanol als Niedrigalkohol bildet sich ein Azeotrop mit einem Siedepunkt von ca. 93°C, das besonders leicht abdestilliert werden kann.

Das über die Kolonne abgetrennte Alkohol/Wasser-Gemisch kann in einem Abscheidebehälter in eine obere alkoholreiche Phase, die in die Kolonne zurückgeführt wird sowie eine untere alkoholarme Phase getrennt werden, die einer getrennten Aufarbeitung zugeführt wird. Auf diese Weise ist gewährleistet, daß das Verfahren mit einem geringen Verlust an Niedrigalkohol auskommt.

An die Zugabe des Glucosesirups schließt sich eine Nachreaktion bei 100 bis 115°C über einen Zeitraum von ca. 0,1 bis 2, vorzugsweise 0,5 bis 1,5 h an, während der sichergestellt wird, daß die Glucose vollständig, d. h. zu mindestens 99 Gew.-% - bezogen auf die Einsatzmenge - abreagiert. Das Reaktionsende kann auch durch den Anstieg der Temperatur in der Destillationskolonne erkannt werden, sobald anstelle des Wasser/Alkoholazeotrops nur noch reiner Niedrigalkohol verdampft wird.

### Gewerbliche Anwendbarkeit

Die nach dem erfindungsgemäßen Verfahren hergestellten Niedrigalkyloligoglucoside werden in kurzen Reaktionszeiten erhalten, weisen einen Polyglucosegehalt von weniger als 2, vorzugsweise weniger als 1 Gew.-% - bezogen auf den Feststoffgehalt des Produktes - auf und zeichnen sich durch gute Farbqualität aus.

Sie eignen sich z. B. als Zwischenprodukte zur Herstellung von längerkettigen Alkyloligoglucosiden für Wasch-, Spül- und Reinigungsmittel sowie für Produkte zur Haar- und Körperpflege, in denen sie in Mengen von 0,1 bis 50, vorzugsweise 1 bis 25 Gew.-% - bezogen auf die Mittel - enthalten sein können.

Die folgenden Beispiele sollen den Gegenstand der Erfindung näher erläutern, ohne ihn darauf einzuschränken.

### Beispiele

### Beispiel 1:

In einem 4-l-Rührreaktor wurden 2000 g (27 mol) n-Butanol und 12,7 g p-Toluolsulfonsäure vorgelegt und bei einem Druck von 750 mbar auf 107°C erhitzt. Die Reaktionsmischung wurde über eine außerhalb des Reaktors befindliche Mischkammer, die mit einem Mischer vom Typ Ultra-Turrax(^{R}) ausgerüstet war, im Kreis gepumpt. Innerhalb von 2 h wurden 1160 g Glucosesirup (Feststoffgehalt 70 Gew.-%, Glucosegehalt : 96 Gew.-% bezogen auf Feststoffanteil) - entsprechend 4,5 mol Glucose - über die Mischkammer des Ultra-Turrax(^{R}) in den Flüssigkeitskreislauf eindosiert, wobei eine feine Dispersion der Glucose im Butanol entstand.

Das Reaktionswasser sowie Wasser, das über den Glucosesirup eingebracht worden war, wurde in Form eines Butanol/Wasser-Azeotrops abdestilliert.

Das Kondensat wurde in einem Abscheider getrennt und die butanolreiche Phase in den Reaktor zurückgeführt.

Nach Beendigung der Zugabe wurde die Reaktionsmischung über einen Zeitraum von 1 h bei 107°C einer Nachreaktion unterworfen.

Die Versuchsergebnisse sind in Tab.1 zusammengefaßt.

### Beispiel 2:

In einem 3,2 m³-Reaktor wurden 870 kg Butanol und 4,3 kg p-Toluolsulfonsäure vorgelegt und bei einem Druck von 950 mbar auf 110°C erhitzt. Über eine Pumpe, einen Inline-Mischer (Typ Cavitron(^{R}) 1039, Drehzahl 3100 UpM) und einen Fallfilmverdampfer wurde ein Flüssigkeitskreislauf aufgebaut. Innerhalb von 3 h wurden 470 kg Glucosesirup (Feststoffgehalt : 70 Gew.-%, Glucosegehalt : 96 Gew.-% bezogen auf den Feststoffgehalt) in den Inline-Mischer dosiert.

Das Reaktionswasser sowie Wasser, das über den Glucosesirup eingebracht worden war, wurde in Form eines Butanol/Wasser-Azeotrops abdestilliert.

Das Kondensat wurde in einem Abscheider getrennt und die butanolreiche Phase in den Reaktor zurückgeführt.

Nach Beendigung der Zugabe wurde die Reaktionsmischung über einen Zeitraum von 1 h bei 110°C einer Nachreaktion unterworfen. Die Versuchsergebnisse sind in Tab.1 zusammengefaßt.

### Vergleichsbeispiel V1:

Beispiel 1 wurde wiederholt, der Glucosesirup jedoch nicht über den Ultra-Turrax(^{R}), sondern innerhalb von 2 h direkt in den Reaktor eindosiert. Anstelle einer feinen Dispersion wurde beobachtet, daß sich der Glucosesirup in der Reaktionsmischung kaum verteilte, sondern an der Wandung des Reaktors und insbesondere an der Rührvorrichtung abschied. Die Nachreaktionszeit verlängerte sich auf 2,5 h. Infolge von partieller Verkohlung des Glucosesirups an der Reaktorwand fanden sich schwarze Feststoffpartikel im Produkt. Die Ergebnisse sind in Tab.1 zusammengefaßt.

### Vergleichsbeispiel V2:

Beispiel 2 wurde wiederholt, der Glucosesirup jedoch nicht über den Inline-Mischer, sondern innerhalb von 2 h direkt in den Reaktor eindosiert. Wiederum wurde keine feine Dispersion erhalten und das Verkleben von Rührer und Reaktorwandung beobachtet. Die Ergebnisse sind in Tab.1 zusammengefaßt.

**Tab.1:**

| Versuchsergebnisse Prozentangabe als Gew.-% | | | | | |
|---|---|---|---|---|---|
| Bsp. | t(D) h | t(NRk) h | Dispersion | Produkt | c(PG) % |
| 1 | 2 | 1 | feinteilig | hellfarbig, klar | < 1 |
| 2 | 2 | 1 | feinteilig | hellfarbig, klar | < 1 |
| V1 | 2 | 2,5 | klumpig | verfärbt, Verkohlungen | 2,1 |
| V2 | 2 | 3 | klumpig | verfärbt, Verkohlungen | 2,5 |
| Legende: t(D) = Dosierzeit t(NRk) = Nachreaktionszeit c(PG) = Gehalt Polyglucose | | | | | |

## Patentansprüche

1. Verfahren zur Herstellung von Niedrigalkyloligoglucosiden, **dadurch gekennzeichnet**, daß man
a) wäßrigen Glucosesirup bei erhöhten Temperaturen über einen Inline-Mischer einer Mischung aus einem Niedrigalkohol und einem sauren Katalysator zudosiert,
b) im Reaktionsgemisch enthaltenes sowie freigesetztes Wasser kontinuierlich azeotrop abdestilliert sowie
c) die Reaktionsmischung nach Beendigung der Zugabe an Glucosesirup einer Nachreaktion unterwirft.

2. Verfahren nach Anspruch 1, **dadurch gekennzeichnet**, daß man Glucosesirup mit einem Feststoffanteil von 50 bis 85 Gew.-% - bezogen auf den Sirup - einsetzt.

3. Verfahren nach den Ansprüchen 1 und 2, **dadurch gekennzeichnet**, daß man Glucosesirup mit einem DP-1 Gehalt von 90 bis 100 Gew.-% - bezogen auf den Feststoffanteil - einsetzt.

4. Verfahren nach mindestens einem der Ansprüche 1 bis 3, **dadurch gekennzeichnet**, daß man Inline-Mischer mit einem Schergefälle von 10⁴ bis 10⁶ s⁻¹ einsetzt.

5. Verfahren nach mindestens einem der Ansprüche 1 bis 4, **dadurch gekennzeichnet**, daß man Inline-Mischer mit einer mittleren Scherimpulszahl pro Volumeneinheit von 10⁵ bis 10⁸ l⁻¹ einsetzt.

6. Verfahren nach mindestens einem der Ansprüche 1 bis 5, **dadurch gekennzeichnet**, daß man Niedrigalkohole der Formel (I) einsetzt,
**R**^{**1**}**-OH** (I)
in der R¹ für einen linearen oder verzweigten Alkylrest mit 3 bis 8 Kohlenstoffatomen steht.

7. Verfahren nach mindestens einem der Ansprüche 1 bis 6, **dadurch gekennzeichnet**, daß man saure Katalysatoren einsetzt, die ausgewählt sind aus der Gruppe, die von p-Toluolsulfonsäure, Methansulfonsäure, Butansulfonsäure und Sulfobernsteinsäure gebildet wird.

8. Verfahren nach mindestens einem der Ansprüche 1 bis 7, **dadurch gekennzeichnet**, daß man Glucose und Niedrigalkohol in einem molaren Verhältnis von 1 : 3 bis 1 : 10 einsetzt.

9. Verfahren nach mindestens einem der Ansprüche 1 bis 8, **dadurch gekennzeichnet**, daß man den sauren Katalysator in Mengen von 3 * 10⁻³ bis 2 * 10⁻² mol pro mol Glucose einsetzt.

10. Verfahren nach mindestens einem der Ansprüche 1 bis 9, **dadurch gekennzeichnet**, daß man den Glucosesirup über einen Zeitraum von 0,1 bis 3 h mit einer solchen Geschwindigkeit zudosiert, daß eine feinteilige Dispersion entsteht.

11. Verfahren nach mindestens einem der Ansprüche 1 bis 10, **dadurch gekennzeichnet**, daß man die Nachreaktion über einen Zeitraum von 0,1 bis 2 h durchführt.

12. Verfahren nach mindestens einem der Ansprüche 1 bis 11, **dadurch gekennzeichnet**, daß man die Acetalisierung und die Nachreaktion bei Temperaturen von jeweils 100 bis 115°C durchführt.

## Claims

1. A process for the production of lower alkyl oligoglucosides, characterized in that
a) aqueous glucose sirup is added to a mixture of a lower alcohol and an acidic catalyst at elevated temperatures **via** an inline mixer,
b) the water present in the reaction mixture and the water released are azeotropically distilled off continuously and
c) after the addition of glucose sirup, the reaction mixture is subjected to an after-reaction.

2. A process as claimed in claim 1, **characterized in that** glucose sirup having a solids content of 50 to 85% by weight, based on the sirup is used.

3. A process as claimed in claims 1 and 2, **characterized in that** glucose sirup having a DP 1 content of 90 to 100% by weight, based on the solids content, is used.

4. A process as claimed in at least one of claims 1 to 3, **characterized in that** inline mixers having a shear rate of 10⁴ to 10⁶ s⁻¹ are used.

5. A process as claimed in at least one of claims 1 to 4, **characterized in that** inline mixers having an average shear pulse count per unit volume of 10⁵ to 10⁸ l⁻¹ are used.

6. A process as claimed in at least one of claims 1 to 5, **characterized in that** lower alcohols corresponding to formula (I)
**R**^{**1**}**-OH** **(I)**
in which R¹ is a linear or branched alkyl radical containing 3 to 8 carbon atoms are used.

7. A process as claimed in at least one of claims 1 to 6, **characterized in that** acidic catalysts selected from the group consisting of p-toluenesulfonic acid, methanesulfonic acid, butanesulfonic acid and sulfosuccinic acid are used.

8. A process as claimed in at least one of claims 1 to 7, **characterized in that** glucose and lower alcohol are used in a molar ratio of 1:3 to 1:10.

9. A process as claimed in at least one of claims 1 to 8, **characterized in that** the acidic catalyst is used in quantities of 3 · 10⁻³ to 2 · 10⁻² mol per mol glucose.

10. A process as claimed in at least one of claims 1 to 9, **characterized in that** the glucose sirup is added over a period of 0.1 to 3 h at such a rate that a fine-particle dispersion is formed.

11. A process as claimed in at least one of claims 1 to 10, **characterized in that** the after-reaction is carried out over a period of 0.1 to 2 h.

12. A process as claimed in at least one of claims 1 to 11, **characterized in that** the acetalization and the after-reaction are carried out at temperatures of 100 to 115°C.

## Revendications

1. Procédé de préparation d'alkyloligoglucosides inférieurs, **caractérisé en ce que** l'on
a) ajoute progressivement un sirop de glucose aqueux à températures accrues via un mélangeur installé en ligne à un mélange d'un alcool inférieur et d'un catalyseur acide,
b) élimine continuellement par distillation azéotrope l'eau contenue dans le mélange réactionnel ainsi que l'eau libérée et que l'on
c) soumet le mélange réactionnel à une réaction postérieure après la fin de l'adjonction du sirop de glucose.

2. Procédé selon la revendication 1, **caractérisé en ce que** l'on utilise du sirop de glucose avec une proportion de matière solide de 50 à 85 % en poids par rapport au sirop.

3. Procédé selon les revendications 1 et 2, **caractérisé en ce que** l'on utilise du sirop de glucose avec une teneur en DP-1 de 90 à 100 % en poids par rapport à la proportion de matière solide.

4. Procédé selon au moins une des revendications 1 à 3, **caractérisé en ce que** l'on utilise un mélangeur installé en ligne présentant un gradient de cisaillement de 10⁴ à 10⁶ s⁻¹.

5. Procédé selon au moins une des revendications 1 à 4, **caractérisé en ce que** l'on utilise un mélangeur installé en ligne présentant un nombre d'impulsions de cisaillement par unité volumique de 10⁵ à 10⁸ **l**⁻¹.

6. Procédé selon au moins une des revendications 1 à 5, **caractérisé en ce que** l'on utilise des alcools inférieurs de la formule (I),
R¹OH (I)
dans laquelle R¹ représente un radical alkyle linéaire ou ramifié comportant 3 à 8 atomes de carbone.

7. Procédé selon au moins une des revendications 1 à 6, **caractérisé en ce que** l'on utilise des catalyseurs acides, qui sont sélectionnés parmi le groupe constitué des acides p-toluènesulfonique, méthanesulfonique, butanesulfonique et sulfosuccinique.

8. Procédé selon au moins une des revendications 1 à 7, **caractérisé en ce que** l'on met en oeuvre le glucose et l'alcool inférieur dans un rapport molaire de 1:3 à 1:10.

9. Procédé selon au moins une des revendications 1 à 8, **caractérisé en ce que** l'on met en oeuvre le catalyseur acide en proportions de 3 * 10⁻³ à 2 * 10⁻² mole par mole de glucose.

10. Procédé selon au moins une des revendications 1 à 9, **caractérisé en ce que** l'on ajoute progressivement le sirop de glucose au cours d'une période de 0,1 à 3 h à une vitesse telle que l'on obtient une dispersion à fines particules.

11. Procédé selon au moins une des revendications 1 à 10, **caractérisé en ce que** l'on opère la réaction postérieure au cours d'une période de 0,1 à 2 h.

12. Procédé selon au moins une des revendications 1 à 11, **caractérisé en ce que** l'on opère l'acétalisation et la réaction postérieure à des températures de 100 à 115 °C dans chaque cas.
